(19) Europäisches Patentamt
European Patent Office
Office européen des brevets

(11) EP 4 295 695 A1

(12) # EUROPEAN PATENT APPLICATION
published in accordance with Art. 153(4) EPC

(43) Date of publication:
**27.12.2023 Bulletin 2023/52**

(21) Application number: **22756220.4**

(22) Date of filing: **16.02.2022**

(51) International Patent Classification (IPC):
***A23K 20/105*** *(2016.01)* ***A23K 20/142*** *(2016.01)*
***A23K 20/184*** *(2016.01)*

(52) Cooperative Patent Classification (CPC):
**A23K 20/105; A23K 20/142; A23K 20/184;**
Y02P 60/87

(86) International application number:
**PCT/JP2022/006143**

(87) International publication number:
**WO 2022/176902 (25.08.2022 Gazette 2022/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.02.2021 JP 2021023495**

(71) Applicant: **SUMITOMO CHEMICAL COMPANY, LIMITED**
**Tokyo 103-6020 (JP)**

(72) Inventors:
- **KOJO, Hiroshi**
**Tokyo 103-6020 (JP)**
- **TONOUE, Tsuyoshi**
**Tokyo 103-6020 (JP)**
- **IKARI, Kaori**
**deceased (JP)**

(74) Representative: **Vossius & Partner Patentanwälte Rechtsanwälte mbB**
**Siebertstraße 3**
**81675 München (DE)**

(54) **LIVESTOCK FEED**

(57) A feed for livestock is provided that shows excellent body weight gain and improved feed efficiency. A feed additive composition and a feed for livestock comprising abscisic acid and/or a salt thereof and glycine; a method of feeding livestock, a method of improving the weight gain of livestock, and a method of increasing the feed efficiency in livestock, comprising letting livestock to take said feed; and a use of the feed for livestock for improving the weight gain of livestock, and a use of the feed for increasing the feed efficiency in livestock.



Processed by Luminess, 75001 PARIS (FR)

## Description

TECHNICAL FIELD

This application claims priority to and the benefit of Japanese Patent Application No. 2021-023495 filed February 17, 2021, the entire contents of which are incorporated herein by reference.

The present invention relates to a feed for livestock. More particularly, the present invention relates to a feed for livestock comprising abscisic acid (hereinafter also referred to as "ABA") and/or its salts and glycine.

BACKGROUND ART

Livestock are animals that are acclimatized and bred for human use of their products (milk, meat, eggs, hair, skin, fur, labor, etc.) and are bred in human captivity. On the other hand, in recent years, the term "industrial animals" has become widespread, and industrial animals are animals belonging to mammals and birds that are bred or stored for industrial use.

Livestock can be roughly classified into agricultural animals, pets and experimental animals depending on the purpose of use, but in a narrow sense, livestock may refer only to agricultural animals. Agricultural animals are divided into livestock that produce the above products and livestock the labor force of which is utilized.

Livestock are fed on feed for oral nutrient supplementation. Demand for mixed feeds and compound feeds, which are a mixture of various types of feed, is significantly increasing depending on the purpose of breeding the animals to be fed and the breed of livestock. For the main feeds, milo (coryan), corn, oat, wheat, barley, soybean meal, sesame seeds meal, flaxseed meal, cottonseed meal, peanut meal, powdered fish meal, meat meal, blood powder, fish meal, rice meal, dregs, starch meal, etc. have been used.

Feed additives are added by means of addition, mixture, wetting or other methods to the feed for the purpose of preventing deterioration of feed quality, of supplementing the nutritional components of the feed and other active ingredients, and of promoting the effective utilization of the nutritional components contained in the feed. As feed additives for preventing deterioration of feed quality, antioxidants, fungicides, binders, emulsifiers and modifiers are used. As feed additives for supplementing nutritional components and other active ingredients of feeds, vitamins, minerals and color enhancers (carotenoids) are used. As feed additives for promoting the effective utilization of nutritional components contained in the feed, synthetic antibacterial agents, antibiotics, flavoring agents, tasting agents, enzymes and organic acids are used.

Abscisic acid (ABA) is a natural occurring plant hormone, widely present in plants and is a substance that carries out physiological activity and signal transduction between cells.

Abscisic acid is synthesized in cells when plants are exposed to environmental stresses such as dryness and low temperature, and plays a role in drying tolerance, growth suppression, seed dormancy and the like. Commercial formulations containing abscisic acid are used as a so-called plant growth regulator as improving stress tolerance at harvest and at planting time or around them, slowing the growth rate, and regulating the flowering period, etc. in agriculture and horticulture.

Abscisic acid or its' salts or esters is also used in the treatment of vitamin deficiency (Patent document 1), the treatment of diabetes and immune system diseases (Patent document 2), and the treatment of neurodegenerative diseases (Patent document 3). ABA and its biosynthetic precursor xanthoxin are reported to be inhibitors of a human Bitter Taste G-protein Coupled Receptor (Non-patent document 1). ABA metabolite phaseic acid has been shown to be neuroprotective during ischemic brain injury (Non-patent document 2).

An example of using abscisic acid for livestock and fish includes Patent documents 4 to 8. Patent document 4 discloses that, by providing a feed comprising as an effective component abscisic acid, the feed efficiency, the weight gain and the muscle gain of livestock and fish can be improved, a raising period can be shortened, and a dose of antibiotics can be reduced or antibiotics no longer be used.

Patent document 5 discloses a composition comprising abscisic acid, a salt thereof, a derivative thereof and an analogue thereof, and a method of improving post-natal weight gain of animal offspring by using said composition.

Patent document 6 discloses a composition comprising abscisic acid, a salt thereof, a derivative thereof and an analogue thereof, and a method of increasing the feed efficiency comprising administering abscisic acid, a salt thereof, and a derivative thereof to livestock or fish.

Patent document 7 discloses a composition comprising abscisic acid, a salt thereof, a derivative thereof and an analogue thereof, and a method of improving the productivity of animal reproduction comprising administering abscisic acid to a pregnant animal.

Patent document 8 discloses a composition comprising abscisic acid, a salt thereof, a derivative thereof and an analogue thereof, and a method of reducing mortality comprising administering abscisic acid, a salt thereof and a derivative thereof to chicken.

CITATION LIST

PATENT DOCUMENT

Patent Document 1: USP 3958025
Patent Document 2: USP 7741367
Patent Document 3: USP 7718699
Patent Document 4: WO 2012037561
Patent Document 5: USP 9591867
Patent Document 6: US patent publication No. 2015/0250209
Patent Document 7: USP 10238613
Patent Document 8: US patent publication No. 2020/0030269

Non-PATENT DOCUMENT

Non-Patent Document 1: Pydi, et al., Biochemistry, 2015, 54, 2622-2631.
Non-Patent Document 2: Hou, et al., The Journal of Biological Chemistry, 2016, 291, 27007-27022.

SUMMARY OF THE INVENTION

(PROBLEMS TO BE SOLVED BY INVENTION)

As described above, various feed additives have been added to a feed for livestock for the purpose of preventing deterioration of feed quality and the like. There is a need for a feed which efficiently allows for the increase in the body weight of livestock and increases livestock productivity.

(MEANS TO SOLVE PROBLEMS)

As a result of diligent research to solve the above problems, the present inventor has found that a feed containing abscisic acid and/or a salt thereof and glycine is effective in efficiently increasing the weight of livestock in a small amount to thereby complete the present invention.

Thus, the present invention relates to a feed additive composition for livestock comprising abscisic acid and/or a salt thereof and glycine; a feed for livestock comprising abscisic acid and/or a salt thereof and glycine; a method of feeding livestock, a method of improving the weight gain of livestock, and a method of increasing the feed efficiency in livestock, which comprises feeding the livestock with said feed for livestock.

Accordingly, the present invention includes the followings.

[1] A feed additive composition for livestock comprising abscisic acid and/or a salt thereof and glycine.
[2] A feed for livestock comprising abscisic acid and/or a salt thereof and glycine.
[3] The feed for livestock of [2] wherein the feed comprises abscisic acid and/or a salt thereof at 0.01 to 100 ppm.
[4] The feed for livestock of [2] wherein the feed comprises abscisic acid and/or a salt thereof at 0.1 to 10 ppm.
[5] The feed for livestock of [3] wherein a weight ratio of abscisic acid and/or a salt thereof to glycine is 1 : about 30 to about 400,000.
[6] The feed for livestock of [4] wherein a weight ratio of abscisic acid and/or a salt thereof to glycine is 1 : about 300 to about 40,000.
[7] The feed for livestock of any one of [2] to [6] wherein the feed is for mammals.
[8] A method of feeding livestock comprising letting livestock to take the feed of any one of [2] to [7].
[9] A method of improving the weight gain of livestock comprising letting livestock to take the feed of any one of [2] to [7].
[10] A method of increasing the feed efficiency in livestock comprising letting livestock to take the feed of any one of [2] to [7] .
[11] A use of the feed for livestock of any one of [2] to [7] for improving the weight gain of livestock.
[12] A use of the feed for livestock of any one of [2] to [7] for increasing the feed efficiency in livestock.

(EFFECT OF INVENTION)

A feed of the present invention comprises a combination of both abscisic acid and/or a salt thereof and glycine to thereby exert synergistic effects with regard to the body weight gain and the feed efficiency as compared to each alone of abscisic acid and/or a salt thereof and glycine.
In accordance with the present invention, by feeding livestock with a feed comprising a combination of abscisic acid and/or a salt thereof and glycine, the weight gain of livestock is improved and the feed efficiency in livestock can be increased.

MODE FOR CARRYING OUT THE INVENTION

"Abscisic acid and/or a salt thereof" to be added to a feed in accordance with the present invention is preferably (S)-(+)-abscisic acid (hereinafter also referred to as "S-abscisic acid" or "S-ABA"), which is one of enantiomers of abscisic acid, and/or a salt thereof and is most preferably S-ABA. S-abscisic acid has the following structure.

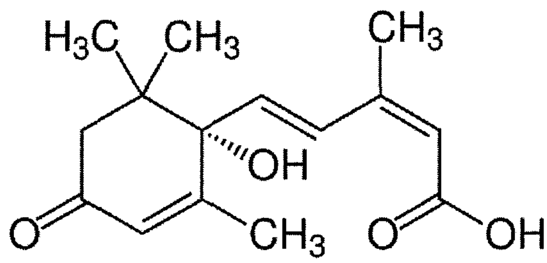

An example of an abscisic acid salt which can be used in the present invention includes an inorganic acid salt, for example, ammonium salts, lithium salts, sodium salts, potassium salts and magnesium salts; and organic acid salts, for example, triethanolamine salts, dimethanolamine salts, and ethanolamine salts. An abscisic acid salt is not limited to these in the present invention and includes any other salts. The preferred salts in the present invention are ammonium salts. Another preferred salts in the present invention are sodium salts and potassium salts. The production of the salt can be carried out in a conventional manner by contacting abscisic acid with a sufficient amount of a desired base. The salts may be treated with an appropriate dilute acid solution, for example, dilute sulfuric acid, hydrochloric acid or phosphoric acid to regenerate free acids. The free acids are different from their respective acids in some physical properties, for example, solubility in a polar solvent, but any forms can be used in the present invention. An abscisic acid salt which can be used in the present invention is a pharmaceutically acceptable salt.
Production of abscisic acid per se is not particularly limited and may be performed by conventional methods. For example, a method using Botrytis bacteria (JP 61-35838) and a method using Cercospora rosicola [Experimenta 33, 1556 (1977), JP 58-36393, JP 56-160996] are known.
Bacteria to be used for the production of ABA are not particularly limited as far as they are abscisic acid-producing bacteria belonging to Botrytis or Cercospora, including normal mutated bacteria and bacteria generated by treatment of mutation. Abscisic acid-producing bacteria belonging to Botrytis include Botrytis cinerea FERM P-6156. Mycological properties of Botrytis cinerea having abscisic acid-producing ability have been studied in JP 61-35838.
As a culture medium used for abscisic acid production, a solid or liquid medium is used. This medium contains carbon sources such as bran, wheat, rice, sweet potato, potato, glucose, maltose, malt extract, sucrose, dextrin, waste sugar-free, starch and the like, and nitrogen sources such as defatted soy flour, soy flour, gluten, yeast extract, peptone , meat extract, corn steep liquor and the like, each alone or in combination of two or more thereof. In addition, inorganic substances such as magnesium salt, potassium salt, sodium salt, phosphate and the like, vitamins, fats and oils, and others can be added.
The culture medium thus obtained is subjected to a sterilization treatment by a conventional method to give a substantially sterile medium, followed by inoculation with a fungus. Sterilized water containing a spore part of abscisic acid-producing bacteria belonging to the genus Botrytis is used for the medium to which the sterilization has been applied and the spores are uniformly dispersed in the medium so as to inoculate the seed spores. In this case, of course, the medium may be contaminated with mycelial parts other than spores. By inoculating and cultivating seed spores, more uniform cultivation can be carried out and abscisic acid can be accumulated at a high concentration at a high speed.
The culture conditions are as follows: the culture temperature is usually 10 to 40°C, preferably 20 to 30°C; the pH of the medium is usually 3 to 12, preferably 4 to 8; the culture period is usually 1 to 30 days, preferably 5 to 15 days. Cultivation is performed using a culture system excluding contamination of germs. It is particularly preferable to carry out aeration-agitation culture. Abscisic acid is also produced in stationary culture, but its production is markedly promoted

by aeration stirring culture.

After completion of the cultivation, ordinary methods can be adopted for isolating abscisic acid from the medium, for example, the method described below is used. First, cells are removed from the medium by centrifugation, the supernatant is adsorbed on activated carbon, and eluted with an organic solvent. As the elution solvent in this case, for example, a solvent such as acetone, methanol, ethanol or the like is used. Abscisic acid transferred into the eluate can be isolated and purified from the mixture after cultivation by applying general purification methods such as ordinary fractionation extraction, adsorption, partitioning, thin layer chromatography, distillation and the like and ordinary purification methods for organic compound.

A feed for livestock of the present invention is characterized by that the feed comprises glycine in combination with abscisic acid and/or its salts. By the presence of glycine in combination with abscisic acid and/or its salts, synergistic effects with regard to the body weight gain and the feed efficiency can be obtained as compared to a feed comprising either abscisic acid and/or a salt thereof or glycine each alone. Hereinafter, a feed for livestock of the present invention is also referred to as "an ABA mixture feed".

An ABA mixture feed of the present invention can be prepared by mixing a feed for livestock as commonly used (hereinafter also referred to as "a basal feed") with both abscisic acid and/or its salts and glycine. For preparing an ABA mixture feed of the present invention, a feed additive composition for livestock comprising abscisic acid and/or its salts and glycine may be mixed with a basal feed, or alternatively, each of abscisic acid and/or its salts and glycine may be mixed with a basal feed. Administration of an ABA mixture feed of the present invention to livestock is not particularly limited, but can preferably be done in view of simplicity by feeding livestock with an ABA mixture feed prepared by mixing a basal feed with both abscisic acid and/or its salts and glycine. An amount of abscisic acid and/or its salts to be mixed is preferably in a range of from 0.01 to 100 ppm, and more preferably of from 0.1 to 10 ppm. Preferably about 30 to 400,000, or more preferably about 300 to 40,000, of glycine may be mixed with 1 of abscisic acid and/or its salts. That is, a weight ratio of abscisic acid and/or a salt thereof to glycine is 1 : about 30 to about 400,000, and more preferably 1 : about 300 to 40,000. As such, by mixing a very small amount of abscisic acid and/or its salts with glycine, the effects such as the body weight gain and the increased feed efficiency can be obtained. In particular, by adjusting a mixed ratio of abscisic acid and/or its salts with glycine as mentioned above, more excellent effects with regard to the body weight gain and the increased feed efficiency than expected in case that either abscisic acid and/or its salts or glycine is mixed each alone with a basal feed, so-called synergic effect, can be obtained.

A basal feed for use in admixture with an ABA mixture feed of the present invention is not particularly limited and may be the one which includes at least one of the followings: cereals such as ethiopian oat, foxtail millet, barnyardgrass, wheat (extruded), rice (extruded), soybean (extruded), corn (extruded), pea, oat, barley, sweetened heat processing soybean, sweet potato, roasted soybean flour, millet, cassava, cassava meal, grain sorghum (milo), brown rice, sesame, wheat, wheat flour, finger millet, lowest grade flour, sudan grass, littel millet, polished rice, buckwheat, fava bean, soybean, dehulled lupine, teosint, dextrin, pearl millet, corn, cornstarch, velvet bean, naked barley, potato starch, bread crumb, Japanese millet, chick pea, soybean (dry heat), de hulled soybean (dry heat), rice hull, sorghum, rye and lupine; bran such as millet bran, barley shochu distillers residue, barley distillers grain, barley distillers grain with soluble, barley distillers soluble, guar meal, citric acid fermentation by-product, glycine fermentation by-product, dehulled rice disitillers grain, dehulled rice disitillers grain with soluble, wheat, dehulled rice disitillers grain, wheat distillers grain, wheat disaster grain with soluble, wheat, corn distillers grain with soluble, rice shochu distillers residue, rice bran, rice germ, corn gluten feed, sake cake, shochu cake, soy sauce cake, wheat screening pellets, polished rice · wheat distillers grain, polished rice · wheat · brown sugar solution distillers grain, polished rice distillers grain with soluble, soybean hull, beet sugar solution · wheat distillers grain, beet sugar solution distillers grain, sweet potato pulp, cassava pulp, potato pulp, molasses distillers solubles, corn · barley distillers grain, corn · barley distillers grain with soluble, corn - barley distillers soluble, corn distillers grain, corn distillers grain with soluble, corn distillers soluble, malt sprout, brewers grain, wheat bran, wheat bran (exploded), hominy feed, barley bran with hull, barley bran with hull and polish, barley bran with polish, naked barley bran with hull and polish, and lysine fermentation by-product; vegetable oil cakes such as oil coconut sky bunch extract, linseed meal, soybean meal (extruded), dehulled soybean meal (extruded), rapeseed meal (extruded), pea protein, soybean meal (steamed and cooked), dehulled soybean meal (extruded and browned with corn syrup), rapeseed meal (browned with corn syrup), soybean meal (browned with corn syrup), kapok seed meal, soybean meal (bean refuse, press extracted), sesame meal, wheat gluten, wheat gluten (degraded with enzyme), corn gluten meal, corn germ meal, sunflower meal, dehulled sunflower meal, soybean meal, dehulled soybean meal, soybean germ meal, soybean whey, rapeseed meal, rice protein concentrate, soybean protein concentrate, dehulled soybean meal (fermented), dehulled soybean meal (degraded with enzyme), potato protein, palm kernel meal, sunflower meal, isolated soybean protein, dehulled soybean meal (exploded), cottonseed meal, copra meal, and groundnut meal ; animal feeds such as shrimp meal, krill meal, poultry by-product meal, whole chicken meal, casein, crab meal, dried pupa, dried whey, fish meal, white fish meal, egg powder, plasma protein concentrate, blood meal, fish protein (degraded with enzyme),dried swine intestine (degraded with enzyme), pupa meal, gelatin, whole egg (degraded with enzyme), dried skim milk, porcine meat and bone meal, whey protein concentrate, fish soluble, fish soluble adsorbed feed, feather meal,

and swine blood protein (hydrolyzed); and other feeds such as flax stem, linseed oil calcium soap, starch syrup waste, alfalfa, alfalfa meal, sainfoin, L-lactic acid, L-lysine hydrochloride, spring vetch, cacao husks, confectionery waste, confectionery bread waste, fructose, turnips, sugar cane top, mushroom bed meal (dry), cassava stem and leaf meal, bermudagrass, fish oil ester, leucaena stem and leaf meal, kudzu, grass pea, glycine by-product, clover, chlorella, mulberry leaf meal, Chinese vetch, yeast extract, coconut milk residue, coffee extract residue, corn cob meal, corn steep liquor, devils tongue flour refuse, sugar, silkworm manure, diureido isobutane, fatty acid calcium, Shea nut meal, food by-product, vegetable oil saponified product, vegetable oil gum, vegetable cooking oil, torula yeast (nucleic acid extracted), torula yeast, bread yeast, brewer's yeast, refined fish meal, noodle waste, soybean germ, soybean oil cake, purple crown vetch, medium chain fatty acid calcium salt, tempura scrap meal, corn germ, DL-methionine, beet sugar stem and leave, beet sugar refinery by-product, soybean milk residue, soybean curd residue, animal fat, molasses, trehalose, rapeseed oil cake, hairy vetch, black locust leaf meal, soy sauce cake and tofu cake (lactic fermented), lactose, urea, tropical kudzu, pineapple bran, bagasse, pasta waste, soybean curd residue (fermented), beet meal, beet pulp, fescue (Festuca), winery waste, glucose, bluegrass, bromegrass (Bromus), mangel beet, orange peelings, orange juice pulp, trefoil, sericea lespedoza, cottonseed, cottonseed hull, kraft pulp, rice hull, wild cabbage, L-lysine sulfate, apple juice pulp, rutabaga, lupine hull, which may be appropriately used in admixture. The poultry feed for use in the present invention preferably contains at least one selected from the group consisting of corn, wheat, milo, oat, barley, soybean, soybean oil cake, rapeseed oil cake, corn distiller's grain solubles, corn gluten feed, bran and rice bran.

An ABA mixture feed of the present invention may further contain vitamins, minerals, guanidinoacetic acid, and the like.

The vitamins include L-ascorbic acid, calcium L-ascorbate, sodium L-ascorbate, L-ascorbic acid-2-phosphoestermagnesium, acetomenaphthone, inositol, dibenzoyl thiamine hydrochloride, ergocalciferol, choline chloride, thiamine hydrochloride, pyridoxine hydrochloride, β-Carotene, cholecalciferol, DL-α-Tocopherol Acetate, retinyl acetate, cyanocobalamin, thiamine mononitrate, nicotinic acid, nicotinamide, p-Aminobenzoic Acid, retinyl palmitate, calcium D-pantothenate, calcium DL-pantothenate, d-biotin, vitamin A Powder, vitamin A Oil, vitamin D Powder, vitamin D3 Oil, vitamin E Powder, 25-hydroxycholecalciferol, retinyl propionate, menadione dimethylpyrimidinol bisulfite, menadione sodium bisulfite, folic acid, riboflavin, riboflavin tetrabutyrate, and the like.

The minerals include zinc chloride, potassium chloride, ferric chloride, copper chloride, copper oxychloride, ferric citrate, ferric ammonium citrate, calcium gluconate, iron and sodium succinate citrate, zinc acetate, cobalt acetate, copper acetate, zinc oxide, copper oxide, magnesium oxide, aluminum hydroxide, manganese hydroxide, selenium, zinc carbonate, cobaltous carbonate, sodium bicarbonate, ferrous carbonate, magnesium carbonate, manganese carbonate, Zn bis(2-hydroxy-4-metylthio butyrate), ferrous DL-threonate, calcium lactate, ferrous fumarate, zinc peptide, iron peptide, copper peptide, manganese peptide, molybdenum, potassium iodide, potassium iodate, calcium Iodate, zinc sulfate, zinc methionine sulfate, sodium sulfate, magnesium sulfate, cobaltous sulfate, ferrous sulfate, copper sulfate, magnesium sulfate, lysine copper complex, potassium hydrogen phosphate, sodium hydrogen phosphate, tricalcium phosphate, potassium dihydrogen phosphate, sodium dihydrogen sulfate, and the like.

An ABA mixture feed of the present invention may further contain a flavoring agent, a taste imparting agent, an enzyme, an organic acid, and the like.

The flavoring agent includes esters, ethers, ketones, fatty acids, aliphatic higher alcohols, aliphatic higher aldehydes, aliphatic higher hydrocarbons, terpene hydrocarbons, phenol ethers, phenols, aromatic alcohols, aromatic aldehydes, lactones, and the like.

The taste imparting agent includes saccharin sodium, and the like.

The enzyme includes amylase, alkaline Protease, galactosidase, xylanase, xylanase and pectinase complex, β-glucanase, acid protease, cellulase, cellulase, protease and pectinase complex, neutral protease, phytase, mannanase, lactase, lipase, and the like.

The organic acid includes calcium formate, sodium gluconate, potassium diformate, fumaric acid, and the like.

An ABA mixture feed of the present invention may be used in combination with synthetic antibacterial agents and antibiotics.

The synthetic antibacterial agents include amprolium plus ethopabate, amprolium plus ethopabate and sulfaquinoxaline, robenidine hydrochloride, morantel citrate, diclazuril, decoquinate, nicarbazin, halofuginone hydrobromide, calcium halofuginone polystyrenesulfonate, and the like.

The antibiotics include Zinc Bacitracin, avilamycin, alkyltrimethylammonium calcium oxytetracycline, enramycin, chlortetracycline, salinomycin sodium, semduramicin sodium, narasin, nosiheptide, bicozamycin, flavophospholipol, maduramycin ammonium, monensin sodium, lasalocid sodium, tylosin phosphate, and the like.

An ABA mixture feed of the present invention may further contain an antioxidant, a binder, an emulsifier, a regulator, etc. in order to prevent deterioration of feed quality.

The antioxidant includes ascorbic acid, sodium ascorbate, calcium ascorbate, α-tocopherol, ethoxyquin, dibutylhydroxy-toluene, ascorbyl palmitate, butylhydroxyanisol, and the like.

The binder includes sodium alginate, sodium caseinate, sodium carboxylmethyl cellulose, propylene glycol,

sodium polyacrylate, and the like.

The emulsifier includes glycerin fatty acid ester, sucrose fatty acid ester, sorbitan fatty acid ester, polyoxyethylene sorbitan fatty acid ester, polyoxyethylene glycerol fatty acid ester, and the like.

A regulator includes formic acid, and the like.

In addition to the above, an ABA mixture feed of the present invention may be used in admixture or in combination with herbs such as, for example, sweet flag (Acorus calamus), garlic (Allium sativum), dill (Anethum graveolens), wormwood (Artemisia absinthium), caraway(Carum carvi), Cinnamomum (Cinnamomum), coriander (Coriandrum sativum), cumin (Cuminum cyminum), turmeric (Curcuma longa), lemon grass (Cymbopogon citratus), artichoke (Cynara scolymus), Echinacea (Echinacea), cardamon (Elettaria cardamomum), fennel (Foeniculum vulgare), ginkgo (Ginkgo biloba), licorice (Glycyrrhiza glabra), common Saint John's wort (Hypericum perforatum), bay laurel (Laurus nobilis), lovage (Levisticum officinale), tea tree (Melaleuca alternifolia), lemon balm (Melissa officinalis), spearmint (Mentha spicata), peppermint (Mentha × piperita), nutmeg (Myristica fragrans), basil (Ocimum basilicum), marjoram (Origanum majorana), oregano (Origanum vulgare), chinese ginseng (Panax ginseng), parsley (Petroselinum sativum), allspice (Pimenta dioica), anise (Pimpinella anisum), pepper (Piper nigrum), patchouli (Pogostemon cablin), rosemary (Rosmarinus officinalis), sage (Salvia officinalis), sweetleaf (Stevia rebaudiana), clove (Syzygium aromaticum), Tansy (Tanacetum vulgare), dandelion (Taraxacum officinale), thyme (Thymus), ajwain (Trachyspermum ammi), ginger (Zingiber officinale), and the like, each alone in combination thereof. Among the herbs mentioned above, sweet flag (Acorus calamus), garlic (Allium sativum), wormwood (Artemisia absinthium), Cinnamomum (Cinnamomum), Echinacea (Echinacea), oregano (Origanum vulgare), clove (Syzygium aromaticum) and thyme (Thymus) are preferably used. The above-mentioned herbs, not only in the form of powders, can be extracted and used in the form of essential oil.

An ABA mixture feed of the present invention may be formulated to tablets, powders, granules, capsules, water-soluble, solutions, water-dispersible or suspensions by adding excipients such as solid carriers and liquid carriers or water. Excipients include gum arabic, albumin, ethylcellulose, kaolin, casein, vital gluten, carrageenan, caramel, carnauba wax, hydrated silicon dioxide, amorphous silicon oxide hydrate, liver powder, agar, xanthan gum, chitosan, roasted soybean flour, guar gum, glycerin, glucomannan, gluten, gluten meal, silicic acid, calcium silicate, magnesium silicate, light anhydrous silicic acid, light liquid paraffin, diatomite, hydrogenated oil, highly saturated fatty acid, wheat flour, wheat middlings, rice bran, rice bran oil cake, corn grits, corn gluten feed, corncob meal, corn starch, shiitake bed log powder, distillers grain, distillers grain soluble, fatty acid, calcium fatty acid, salt, vegetable fat and oil, calcium stearate, zeolite, gelatin, cellulose, soybean mill run, sorbitol, defatted fish meal, powdered skim milk, calcium carbonate, soybean oil cake, soybean hulls, soybean meal, tamarind seed polysaccharide, talc, sodium carbonate, dextran, dextrin, natural aluminum silicate, starch, $\alpha$-starch, animal fat and oil, corn flour, tragacanth gum, torela yeast, actose, concentrated soybean protein, maltose, sucrose, vermiculite, baker's yeast, brewer's yeast, furcellaran, wheat bran, glucose, pullulan, pectin, modified starch for food, bentonite, potato pulp, white fish meal, D-mannitol, anhydrous silicic acid, anhydrous silicates, rice hulls, rice hull powder, calcium lignosulfonate, sodium lignosulfonate, liquid paraffin, calcium hydrogen phosphate, tricalcium phosphate, calcium dihydrogen phosphate, lecithin, locust bean gum, and the like.

An ABA mixture feed of the present invention can be used as a feed for livestock to gain the body weight of livestock and to improve feed efficiency. Also, an ABA mixture feed of the present invention can improve the productivity of livestock reproduction.

As used herein, "daily body weight gain" is a unit which shows a growth rate (an increased amount of body weight) of livestock and is generally indicated as gram/day. It is also called as an average weight gain per day or daily weight gain.

As used herein, "feed conversion ratio (FCR)" means an intake (or consumption) of feed necessary for the production of 1 kg of livestock product and is an inverse of feed efficiency, indicated by the following formula:

Feed conversion rate = [Feed intake (or Feed consumption) (kg) / Amount of livestock product (kg)]

On the other hand, as used herein, "feed efficiency" means "Amount of livestock product (kg) / Feed intake (or Feed consumption) (kg)". The "amount of livestock product" is the weight gained during the raising period, and is also referred to as "body weight gain".

As used herein, "livestock" refers to animals bred in human captivity, including, but not limited to, mammals such as cows, Banteng, yaks, buffalos, sheep, goats, Arabian camel, Bactrian camel, llama, alpaca, reindeer, pigs, horses, donkeys, dogs, minks, ferrets, cats, hamsters, mice, rats, guinea pigs, and rabbits; birds (poultry) such as chickens, quails, turkey, guinea fowl, pigeons, ducks, cairina moschata, geese, and canaries; fish such as carp and goldfish; insects such as silkworm and bees. An ABA feed mixture of the present invention is preferably used as a feed for mammals such as cows, pigs, sheep, goats and horses, more preferably as a feed for monogastric mammals, and

most preferably as a feed for pigs.

The ABA mixture feed of the present invention is preferably used as a feed for mammals such as cattle, pigs, sheep, goats, and horses, more preferably as a feed for monogastric mammals, particularly preferably as a feed for pigs.

## EXAMPLES

The present invention is explained in more detail with the following examples but is not limited thereto. In the following examples, rats were used to study the effects of addition of ABA and glycine to a feed. However, the present invention is not limited thereto and the same effects can be obtained with a feed for the other mammals.

### Example 1

The effects of addition of ABA and glycine to a feed were verified as follows:

(1) Materials and methods

For each experiment, two SD female rats of 7 weeks old were used. Two rats per cage were accommodated. The rats were bred by free intake with the feeds of the composition as shown in Table 1 three times per week for three weeks.

[Table 1]

| No. | Basal feed (part) | Glycine (part) | ABA (ppm) |
|---|---|---|---|
| 1-1 | 100 | 0 | 0 |
| 1-2 | 100 | 0 | 10 |
| 1-3 | 100 | 0.312 | 0 |
| 1-4 | 100 | 0.312 | 10 |

(2) Observation and items to be measured

(i) Clinical symptoms: observed daily.
(ii) Body weight: Body weight was measured for each individual three times a week and individual body weight gain was calculated.
(iii) Feed intake: Residual feed and an amount of feeding were measured for each cage three times a week. Daily average feed intake per animal was calculated with the following formula.

[(Previous amount of feeding)-(Residual feed)] / [(Number of rat per cage) x (period of time)]

(Clinical symptoms)

No abnormality was seen in all individuals.

(Weight gain)

The results of weight gain after breeding for 17 days are shown in Table 2. Increased weight gain was obtained in Experiment with a basal feed supplemented with abscisic acid alone (No. 1-2), Experiment with a basal feed supplemented with glycine alone (No. 1-3) and Experiment with a basal feed supplemented with glycine and abscisic acid (No. 1-4) as compared to Experiment with a basal feed alone (No. 1-1). Besides, more weight gain was observed in Experiment with a basal feed supplemented with glycine and abscisic acid (No. 1-4) than expected from the results of No.1-2 and No.1-3. In particular, comparing Experiment with a basal feed supplemented with glycine alone (No. 1-3) and Experiment with a basal feed supplemented with glycine and abscisic acid (No. 1-4), more weight gain was observed in the latter

than the former, demonstrating that prominent effects of weight gain can be obtained by further adding abscisic acid in addition to glycine to a basal feed in accordance with the present invention. Namely, it was confirmed that synergistic effect was exerted by combining glycine with a quite small amount of abscisic acid.

[Table 2]

| No. | Weight gain (g) | Difference from No.1-1 (g) |
|---|---|---|
| 1-1 | 45.0 | - |
| 1-2 | 48.5 | 3.5 |
| 1-3 | 48.5 | 3.5 |
| 1-4 | 60.5 | 15.5 |

(Feed conversion ratio (FCR))

The results of feed conversion ratio after breeding for 17 days are shown in Table 3. Feed conversion ratio is feed intake necessary for increasing 1 kg body weight and is indicated as feed intake divided by weight gain. Decreased feed conversion ratio was observed in Experiment with a basal feed supplemented with abscisic acid alone (No. 1-2), Experiment with a basal feed supplemented with glycine alone (No. 1-3) and Experiment with a basal feed supplemented with glycine and abscisic acid (No. 1-4) as compared to Experiment with a basal feed alone (No. 1-1). In particular, comparing Experiment with a basal feed supplemented with glycine alone (No. 1-3) and Experiment with a basal feed supplemented with glycine and abscisic acid (No. 1-4), more significant reduction in feed conversion ratio was observed in the latter than the former, demonstrating that prominent effects of improved feed efficiency can be obtained by further adding abscisic acid in addition to glycine to a basal feed in accordance with the present invention. Namely, it was confirmed that synergistic effect was exerted by combining glycine with a quite small amount of abscisic acid.

[Table 3]

| No. | FCR | Difference from No.1-1 |
|---|---|---|
| 1-1 | 4.70 | - |
| 1-2 | 4.67 | -0.03 |
| 1-3 | 4.32 | -0.38 |
| 1-4 | 3.74 | -0.96 |

(Production index)

The results of production index after breeding for 17 days are shown in Table 4. Production index is indicated as: (Rearing rate) x (Weight gain/mouse/day) ÷ (Feed conversion ratio). Rearing rate in this experiment is 100%. Thus, production index was calculated as: (Rearing rate; 100) x (Weight gain; Table 2) ÷ (17 days) ÷ (Feed conversion ratio; Table 3). Production index increased in Experiment with a basal feed supplemented with abscisic acid alone (No. 1-2), Experiment with a basal feed supplemented with glycine alone (No. 1-3) and Experiment with a basal feed supplemented with glycine and abscisic acid (No. 1-4) as compared to Experiment with a basal feed alone (No. 1-1). Besides, improvement in production index was observed in Experiment with a basal feed supplemented with glycine and abscisic acid (No. 1-4) than expected from the results of No.1-2 and No.1-3. In particular, comparing Experiment with a basal feed supplemented with glycine alone (No. 1-3) and Experiment with a basal feed supplemented with glycine and abscisic acid (No. 1-4), further improvement in production index was observed in the latter than the former, demonstrating that prominent effects of improvement in production index can be obtained by further adding abscisic acid in addition to glycine to a basal feed in accordance with the present invention. Namely, it was confirmed that synergistic effect was exerted by combining glycine with a quite small amount of abscisic acid.

[Table 4]

| No. | Production index | Difference from No.1-1 |
|---|---|---|
| 1-1 | 5.6 | - |
| 1-2 | 6.1 | 0.5 |

(continued)

| No. | Production index | Difference from No.1-1 |
|---|---|---|
| 1-3 | 6.6 | 1.0 |
| 1-4 | 9.5 | 3.9 |

Example 2

The effects of addition of ABA and glycine to a feed were verified as follows:

(1) Materials and methods

For each experiment, two SD female rats of 7 weeks old were used. Two rats per cage were accommodated. The rats were bred by free intake with the feeds of the composition as shown in Table 5 three times per week for three weeks.

[Table 5]

| No. | Basal feed (part) | Glycine (part) | ABA (ppm) |
|---|---|---|---|
| 2-1 | 100 | 0 | 0 |
| 2-2 | 100 | 0 | 1 |
| 2-3 | 100 | 0.312 | 0 |
| 2-4 | 100 | 0.312 | 1 |

(2) Observation and items to be measured

(i) Clinical symptoms: observed daily.
(ii) Body weight: Body weight was measured for each individual three times a week and individual body weight gain was calculated.
(iii) Feed intake: Residual feed and an amount of feeding were measured for each cage three times a week. Daily average feed intake per animal was calculated with the following formula.

[(Previous amount of feeding)-(Residual feed)] / [(Number of rat per cage) x (period of time)]

(Clinical symptoms)

No abnormality was seen in all individuals.

(Weight gain)

The results of weight gain after breeding for 19 days are shown in Table 6. Increased weight gain was obtained in Experiment with a basal feed supplemented with abscisic acid alone (No. 2-2), Experiment with a basal feed supplemented with glycine alone (No. 2-3) and Experiment with a basal feed supplemented with glycine and abscisic acid (No. 2-4) as compared to Experiment with a basal feed alone (No. 2-1). Besides, more weight gain was observed in Experiment with a basal feed supplemented with glycine and abscisic acid (No. 2-4) than expected from the results of No.2-2 and No.2-3. In particular, comparing Experiment with a basal feed supplemented with glycine alone (No. 2-3) and Experiment with a basal feed supplemented with glycine and abscisic acid (No. 2-4), more weight gain was observed in the latter than the former, demonstrating that prominent effects of weight gain can be obtained by further adding abscisic acid in addition to glycine to a basal feed in accordance with the present invention. Namely, it was confirmed that synergistic effect was exerted by combining glycine with a quite small amount of abscisic acid.

[Table 6]

| No. | Weight gain (g) | Difference from No.2-1 (g) |
|---|---|---|
| 2-1 | 48.0 | - |
| 2-2 | 49.5 | 1.5 |
| 2-3 | 57.5 | 9.5 |
| 2-4 | 64.0 | 16.0 |

(Feed conversion ratio (FCR))

The results of feed conversion ratio after breeding for 12 days are shown in Table 7. Feed conversion ratio is feed intake necessary for increasing 1 kg body weight and is indicated as feed intake divided by weight gain. Decreased feed conversion ratio was observed in Experiment with a basal feed supplemented with abscisic acid alone (No. 2-2), Experiment with a basal feed supplemented with glycine alone (No. 2-3) and Experiment with a basal feed supplemented with glycine and abscisic acid (No. 2-4) as compared to Experiment with a basal feed alone (No. 2-1). Besides, more decreased feed conversion ratio was observed in Experiment with a basal feed supplemented with glycine and abscisic acid (No. 2-4) than expected from the results of No.2-2 and No.2-3. In particular, comparing Experiment with a basal feed supplemented with glycine alone (No. 2-3) and Experiment with a basal feed supplemented with glycine and abscisic acid (No. 2-4), more decreased feed conversion ratio was observed in the latter than the former, demonstrating that prominent effects of decreased feed conversion ratio can be obtained by further adding abscisic acid in addition to glycine to a basal feed in accordance with the present invention. Namely, it was confirmed that synergistic effect was exerted by combining glycine with a quite small amount of abscisic acid.

[Table 7]

| No. | FCR | Difference from No.2-1 |
|---|---|---|
| 2-1 | 5.14 | - |
| 2-2 | 4.56 | -0.58 |
| 2-3 | 4.32 | -0.82 |
| 2-4 | 3.64 | -1.50 |

(Production index)

(Production index)

The results of production index after breeding for 19 days are shown in Table 8. Rearing rate in this experiment is 100%. Thus, production index was calculated as: (Rearing rate; 100) x (Weight gain; Table 6) ÷ (19 days) ÷ (Feed conversion ratio; Table 7) . Production index increased in Experiment with a basal feed supplemented with abscisic acid alone (No. 2-2), Experiment with a basal feed supplemented with glycine alone (No. 2-3) and Experiment with a basal feed supplemented with glycine and abscisic acid (No. 2-4) as compared to Experiment with a basal feed alone (No. 2-1). Besides, improvement in production index was observed in Experiment with a basal feed supplemented with glycine and abscisic acid (No. 2-4) than expected from the results of No.2-2 and No.2-3. In particular, comparing Experiment with a basal feed supplemented with glycine alone (No. 2-3) and Experiment with a basal feed supplemented with glycine and abscisic acid (No. 2-4), further improvement in production index was observed in the latter than the former, demonstrating that prominent effects of improvement in production index can be obtained by further adding abscisic acid in addition to glycine to a basal feed in accordance with the present invention. Namely, it was confirmed that synergistic effect was exerted by combining glycine with a quite small amount of abscisic acid.

[Table 8]

| No. | Production index | Difference from No.2-1 |
|---|---|---|
| 2-1 | 4.9 | - |
| 2-2 | 5.7 | 0.8 |

(continued)

| No. | Production index | Difference from No.2-1 |
|---|---|---|
| 2-3 | 7.0 | 2.1 |
| 2-4 | 9.3 | 4.4 |

INDUSTRIAL APPLICABILITY

A feed for livestock of the present invention comprising abscisic acid and/or a salt thereof and glycine can be used as a feed for livestock excellent in the body weight gain and the feed efficiency for feeding livestock. In accordance with the present invention, by feeding livestock with a feed comprising abscisic acid and/or a salt thereof and glycine, the weight gain of livestock is improved and the feed efficiency in livestock can be increased.

## Claims

1. A feed additive composition for livestock comprising abscisic acid and/or a salt thereof and glycine.

2. A feed for livestock comprising abscisic acid and/or a salt thereof and glycine.

3. The feed for livestock of claim 2 wherein the feed comprises abscisic acid and/or a salt thereof at 0.01 to 100 ppm.

4. The feed for livestock of claim 2 wherein the feed comprises abscisic acid and/or a salt thereof at 0.1 to 10 ppm.

5. The feed for livestock of claim 3 wherein a weight ratio of abscisic acid and/or a salt thereof to glycine is 1 : about 30 to about 400,000.

6. The feed for livestock of claim 4 wherein a weight ratio of abscisic acid and/or a salt thereof to glycine is 1 : about 300 to about 40,000.

7. The feed for livestock of any one of claims 2 to 6 wherein the feed is for mammals.

8. A method of feeding livestock comprising letting livestock to take the feed of any one of claims 2 to 7.

9. A method of improving the weight gain of livestock comprising letting livestock to take the feed of any one of claims 2 to 7.

10. A method of increasing the feed efficiency in livestock comprising letting livestock to take the feed of any one of claims 2 to 7.

11. A use of the feed for livestock of any one of claims 2 to 7 for improving the weight gain of livestock.

12. A use of the feed for livestock of any one of claims 2 to 7 for increasing the feed efficiency in livestock.

## INTERNATIONAL SEARCH REPORT

International application No.
**PCT/JP2022/006143**

### A. CLASSIFICATION OF SUBJECT MATTER

***A23K 20/105***(2016.01)i; ***A23K 20/142***(2016.01)i; ***A23K 20/184***(2016.01)i
FI: A23K20/105; A23K20/142; A23K20/184

According to International Patent Classification (IPC) or to both national classification and IPC

### B. FIELDS SEARCHED

Minimum documentation searched (classification system followed by classification symbols)

A23K20/105; A23K20/142; A23K20/184

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | US 2018/0184699 A1 (PATAGONIA BIOTECHNOLOGIA S.A.) 05 July 2018 (2018-07-05) paragraphs [0052]-[0068] | 1-12 |
| A | JP 2019-031557 A (VALENT BIOSCIENCES LLC) 28 February 2019 (2019-02-28) entire text, all drawings | 1-12 |

☐ Further documents are listed in the continuation of Box C.
☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 March 2022** | **22 March 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/006143**

| Patent document cited in search report | Publication date (day/month/year) | Patent family member(s) | Publication date (day/month/year) |
|---|---|---|---|
| US 2018/0184699 A1 | 05 July 2018 | CA 2990514 A1<br>page 10, line 4 to page 14, line 15 | |
| JP 2019-031557 A | 28 February 2019 | WO 2012/037562 A2<br>entire text, all drawings<br>CN 103179965 A<br>entire text, all drawings<br>US 8729122 B2<br>entire text, all drawings | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021023495 A
- US 3958025 A
- US 7741367 B
- US 7718699 B
- WO 2012037561 A
- US 9591867 B
- US 20150250209 A
- US 10238613 B
- US 20200030269 A
- JP 61035838 A
- JP 58036393 A
- JP 56160996 A


### Non-patent literature cited in the description


- **PYDI et al.** *Biochemistry,* 2015, vol. 54, 2622-2631
- **HOU et al.** *Journal of Biological Chemistry,* 2016, vol. 291, 27007-27022
- *Experimenta,* 1977, vol. 33, 1556